# EUROPÄISCHE PATENTSCHRIFT

(11) **EP 1 723 937 B1**
(45) Veröffentlichungstag und Bekanntmachung des Hinweises auf die Patenterteilung: **14.01.2009**
(21) Anmeldenummer: 06009296.2
(22) Anmeldetag: 05.05.2006
(51) Int. Cl.: A61F 2/84, A61F 2/01

(54) **Vorrichtung zum Einbringen eines Gegenstandes im Inneren eines Blutgefässes oder des Herzens**
Device for delivering an object into a blood vessel or into the heart
Dispositif pour introduire un élément dans un vaisseau sanguin ou dans le coeur

(30) Priorität: 14.05.2005 DE 102005022423
(43) Veröffentlichungstag der Anmeldung: 22.11.2006
(73) Patentinhaber: Osypka, Peter, Dr.-Ing., 79618 Rheinfelden-Herten (DE)
(72) Erfinder: Osypka, Peter, Dr.-Ing., 79618 Rheinfelden-Herten (DE)
(74) Vertreter: Maucher, Wolfgang

(56) Entgegenhaltungen:
- EP-A- 0 696 447
- WO-A-93/11823
- WO-A-96/38193
- US-B1- 6 506 196

## Beschreibung

Die Erfindung betrifft eine Vorrichtung zum Einbringen und Platzieren oder Applizieren eines Gegenstandes im Inneren eines Blutgefäßes oder des Herzens, beispielsweise zum Zuführen einer Gefäßstütze (stent) oder eines mechanischen Blutfilters oder Käfigs oder eines Verschlusses für eine nicht gewollte Öffnung zwischen der linken und der rechten Vorhofkammer des Herzens, mit einem Zuführkatheter, der im wesentlichen äls Schlauch ausgebildet ist und dessen distales Ende in Gebrauchsstellung an der Stelle mündet, wo der Gegenstand platziert werden soll, und mit einer Einführvorrichtung, mit welcher der Katheter von Außen in ein Blutgefäß einführbar ist, wobei in dem Katheter eine Aufnahme für den Gegenstand vorgesehen ist, die im Inneren des Katheters oder Schlauches verschiebbar ist, wobei die Aufnahme zum Umfassen des Gegenstandes oder hülsenförmig ausgebildet ist und ihre Innenlängshöhlung zur Unterbringung des Gegenstandes während seines Transports zu dem distalen Ende des Katheters dient und die Aufnahme an ihrem distalen Ende eine Öffnung oder einen Ausgang zum Ausschieben oder Ausgeben des Gegenstands aus dieser Aufnahme aufweist.

Eine derartige Vorrichtung ist aus EP 0 696 447 A2, insbesondere Fig. 7 und 8 bekannt, die als nächstliegender Stand der Technik angesehen wird.

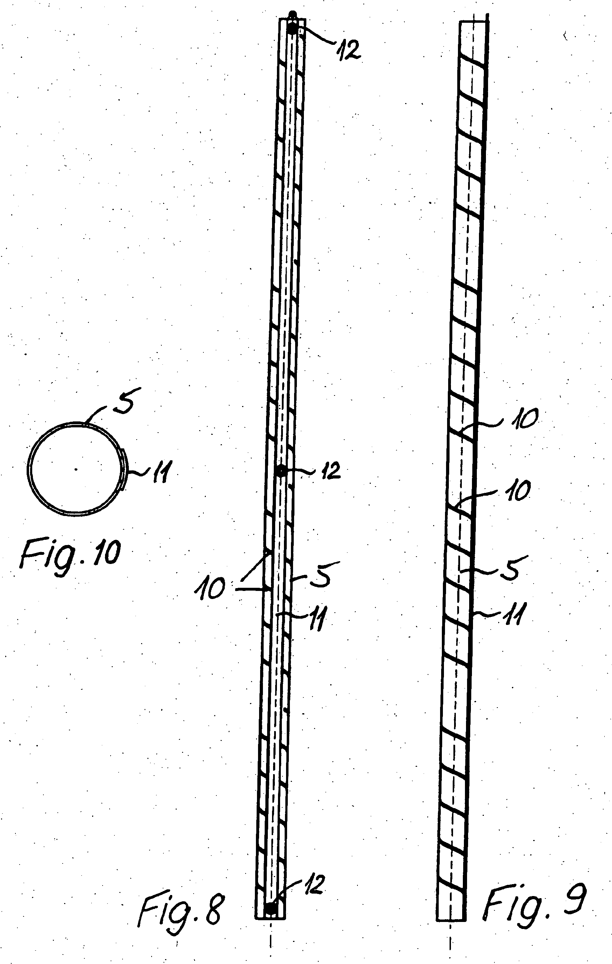

Während andere Ausführungsbeispiele Anordnungen zeigern, bei denen die als Hülle ausgebildet Aufnahme nicht verschiebbar ist und am distalen Ende des Katheters oder Schlauches endet, ist diese hüllenförmige Aufnahme gemäß Fig. 8 um eine Länge (1) gegenüber dem Katheter oder Schlauch verschiebbar.

Dies bedeutet, dass am distalen Ende des Katheters zusätzlicher Raum für wenigstens einen Teil der Aufnahme benötigt wird und darüber hinaus ist nicht sichergestellt, dass die Aufnahme nicht versehentlich zu weit verschoben wird.

Aus US 2002/0013599 A1 ist eine vergleichbare Vorrichtung bekannt, mit welcher ein expandierbarer stent in ein Blutgefäß eingeführt werden soll, welcher dabei von Vorsprüngen innenseitig erfasst ist, die an einem in einem Katheterschlauch verschiebbaren Zuführelemente angeordnet sind. Dabei besteht die Gefahr, dass dieser außenseitig freiliegende stent in Reibkontakt mit dem Züführkatheter gelangt und von diesem Partikel ablöst oder abreibt.

Es besteht deshalb die Aufgabe, eine Vorrichtung der eingangs genannten Art zu schaffen, mit welcher vermieden werden kann, dass ein Gegenstand, beispielsweise ein ASD-Verschluss, bei seiner Beförderung durch den Zuführkatheter von dessen Innenseite, Partikel abreibt und/oder mitnimmt, wobei sichergestellt werden soll, dass die Aufnahme oder das sie bildende Transportgefäß für den zu applizierenden Gegenstand nicht versehentlich aus dem Katheterschlauch herausgeschoben werden kann.

Zur Lösung dieser Aufgabe ist die eingangs definierte Vorrichtung dadurch gekennzeichnet, dass die Aufnahme bis zu dem distalen Ende des Katheters oder Schlauches verschiebbar ist und dass am distalen Ende des Katheters oder Schlauches ein sich radial nach innen erstreckender Anschlag für die Aufnahme vorgesehen ist.

Auf diese Weise kann vermieden werden, dass der einzubringende Gegenstand während seines Transports durch den Zuführkatheter mit dessen Innenseite Berührung hat, so dass er keine Partikel davon ablösen kann und auch von ihm selbst keine Partikel abgerieben werden können. Die Aufnahme kann als Transportgefäß durch den Zuführkatheter beziehungsweise durch den Katheterschlauch verschoben werden und mit dessen Innenseite in Berührung treten, gelangt aber nicht in den Körper, so dass selbst dann, wenn durch diese Verschiebung der.Aufnahme innerhalb des Katheters Partikel gelöst werden sollten, diese nicht in den Körper, in die Blutbahn oder gar in das Herz geraten können.

Durch den erfindungsgemäßen Anschlag für die Aufnahme wird außerdem sichergestellt, dass diese oder das sie bildende Transportgefäß nicht versehentlich aus dem Katheterschlauch herausgeschoben werden kann.

Vor allem eine hülsenförmige und somit eine geschlossene Wandung aufweisende Aufnahme ist günstig, weil die Trennung des einzuführenden Gegenstands von der Innenseite des Katheterschlauches dadurch besonders gut sichergestellt ist.

Dabei kann der Anschlag als an der Stirnseite oder am Innenumfang des Schlauches oder Katheters umlaufender Vorsprung oder Ring ausgebildet sein. Somit wird die Aufnahme zuverlässig an diesem Anschlag angehalten, so dass danach der zu implantierende oder zu applizierende Gegenstand aus ihr herausgeschoben werden kann, wodurch er gleichzeitig auch den Katheterschlauch verlässt.

Der Anschlag kann mit der Mündung des Schlauches oder Katheters bündig sein oder diese Mündung bilden. Somit gelangt das distale Ende der Aufnahme soweit wie möglich an das distale Ende des Katheterschlauches.

Der Katheterschlauch kann aus Metall bestehen und flexibel biegsam ausgebildet sein. Dadurch kann noch besser vermieden werden, dass sich von der Innenseite des Katheters Teile oder Partikel lösen, denn Metall hat eine hohe Festigkeit auch gegen Abrieb, so dass die als Transportgefäß dienende Aufnahme erst recht bei der Bewegung durch diesen metallischen Katheterschlauch beziehungsweise das flexible Metallrohr keine Partikel davon ablösen kann.

Für eine gute Flexibilität des Katheters ist es zweckmäßig, wenn der Katheterschlauch in Umfangsrichtung verlaufende und zueinander beabstandete Schlitze aufweist. Die Schlitze können parallel zueinander angeordnet sein, wobei sie zu einem etwa schraubenlinienförmig verlaufenden Schlitz verbunden oder als Einzelschlitze ausgebildet sein können. Ein derartig gestalteter Katheter kann besonders gut an unterschiedlichste Biegungen oder Krümmungen von Blutgefäßen angepasst werden.

Dabei kann eine ausreichende oder verbesserte Festigkeit des Katheters dadurch erzielt werden, dass oder geschlitzte Schlauch oder Metallschlauch wenigstens einen entlang seiner Erstreckung verlaufenden Stabilisierungsstreifen, insbesondere aus Metall, aufweist, der mit dem Schlauch verbunden, insbesondere verschweißt ist. Bevorzugt ist dabei eine Anordnung dieses Stabilisierungsstreifens an der Außenseite des Katheterschlauches. Dieser Stabilisierungsstreifen kann verhindern, dass der Katheterschlauch insbesondere im Falle von Schlitzungen eventuell in Längsrichtung verformt oder gelängt wird und durch die durch ihn hindurch bewegte Aufnahme beziehungsweise das Transportgefäß in ungewollter Weise verformt werden kann.

Für eine möglichst einfache Abgabe des transportierten Gegenstandes kann zur Verschiebung der Aufnahme ein an dieser direkt oder indirekt angreifender Schieber oder Mandrin vorgesehen sein und dieser Schieber oder Mandrin kann vorzugsweise auch zum Ausschieben des Gegenstands aus der Aufnahme nach Erreichen des Anschlages dienen.

Dabei kann der Mandrin mit dem Gegenstand lösbar verbunden oder verschraubt sein. Somit kann der Verschiebevorgang während des Transports dadurch erfolgen, dass der mit dem Gegenstand lösbar verbundene Mandrin innerhalb, des Schlauches vorgeschoben wird, wodurch gleichzeitig die den Gegenstand enthaltende Aufnahme verschoben wird, bis sie den Anschlag erreicht. Eine weitere Schiebebewegung verursacht dann das Ausschieben des Gegenstands aus der Aufnahme beziehungsweise aus dem Transportgefäß und damit auch aus dem Katheterschlauch. Dabei ist günstig, dass die Reibung zwischen dem Gegenstand und seiner Aufnahme größer als zwischen dieser Aufnahme und dem Katheterschlauch ist.

Damit die Aufnahme auch Krümmungen und Biegungen gut folgen kann, kann die als Aufnahme dienende Hülse eine flexible Wandung haben.

Versuche haben gezeigt, dass es günstig ist, wenn der insbesondere aus Metall bestehende Schlauch des Katheters und/oder die insbesondere hülsenförmige Aufnahme eine Wandstärke von etwa 1/10 mm oder weniger oder etwas mehr haben. Dies ergibt eine verringerte Außenabmessung des Katheterschlauches gegenüber einem Kunststoffschlauch oder erlaubt einen größeren Innenquerschnitt, um einen entsprechend größeren Gegenstand transportieren zu können.

Falls erforderlich, können noch besondere Maßnahmen getroffen sein, um die Reibung zwischen der insbesondere hülsenförmigen Aufnahme und dem Katheterschlauch geringer als zwischen dem Gegenstand und der Aufnahme zu machen. Da jedoch der Gegenstand in der Aufnahme in der Regel zusammengedrückt ist, ergibt sich schon dadurch ein größerer Widerstand dieses Gegenstands gegen sein Herausschieben aus der Aufnahme gegenüber dem Widerstand, den die Aufnahme beim Verschieben innerhalb des Katheterschlauches leistet. Somit bedarf es in der Regel keiner besonderen Maßnahmen, um den Gegenstand in der hülsenförmigen Aufnahme genügend festzulegen, bis diese das distale Ende des Katheterschlauches erreicht hat.

Nachstehend sind Ausführungsbeispiele der Erfindung anhand der Zeichnung näher beschrieben. Es zeigt in zum Teil schematisierter Darstellung:
- Fig. 1: einen in ein Blutgefäß eingeführten und bis in den Bereich der Vorhofkammern des Herzens vorgeschobenen Zuführkatheter, mit welchem ein Gegenstand zum Verschließen einer ungewollten Öffnung zwischen der linken und der rechten Vorhofkammer des Herzens einführbar ist,
- Fig. 2: in vergrößertem Maßstab einen Längsschnitt des Herzens mit einer ungewollten Öffnung im Septum zwischen der linken und der rechten Vorhofkammer, nachdem in diese Öffnung ein ASD-Verschluss eingefügt ist, der durch den Katheterschlauch zugeführt wurde und noch mit einem zum Ausschieben aus dem Schlauch dienenden Mandrin oder Pusher verbunden ist,
- Fig. 3: eine Seitenansicht des Katheters, an dessen proximalen Ende der Mandrin vorsteht,
- Fig. 4: in vergrößertem Maßstab das in Fig.3 durch einen Kreis markierte distale Ende des Katheterschlauches im Längsschnitt, wobei im Inneren des Katheterschlauches eine verschiebbare Aufnahme als Transportgefäß erkennbar ist, die den zu platzierenden Gegenstand in zusammengedrückter Form enthält,
- Fig. 5: eine der Fig.4 entsprechende Darstellung, wobei die Aufnahme einen am distalen Ende des Zuführkatheters befindlichen Anschlag erreicht hat, der ringförmig ausgebildet ist und eine Innenöffnung aufweist, die etwa bündig mit der Innenöffnung der Aufnahme ist, so dass der Gegenstand mit Hilfe des Mandrins - wie dargestellt - durch weitere Vorwärtsbewegung des Mandrins ausschiebbar ist, bis der Gegenstand die in Fig.2 dargestellte Position erreicht hat und sich dort beispielsweise aufgrund seiner Fertigung aus Memory-Material zu einem ASD-Verschluss formt,
- Fig. 6: den Mandrin oder Pusher mit dem zu transportierenden Gegenstand sowie
- Fig. 7: in vergrößertem Maßstab den Mandrin, den zu transportierenden Gegenstand und deren gegenseitige lösbare Verbindung mit Hilfe eines Gewindes, wobei der Gegenstand in der Aufnahme angeordnet ist, die innerhalb des Führungskatheters oder Zuführkatheters verschiebbar ist,
- Fig. 8: eine erste Seitenansicht und
- Fig. 9: eine zweite Seitenansicht eines Zuführkatheters, der zur Vergrößerung seiner Flexibilität mit parallelen, zweckmäßigerweise schraubenlinienförmig verlaufenden Schlitzen versehen und außenseitig durch einen in Längserstreckungsrichtung verlaufenden Streifen stabilisiert ist,
- Fig. 10: im vergrößertem Maßstab einen Querschnitt des Zuführkatheters gemäß Fig.8 und 9,
- Fig. 11: eine erste Seitenansicht und
- Fig. 12: eine zweite Seitenansicht einer abgewandelten Ausführungsform des Zuführkatheters, welcher mit etwa in Umfangsrichtung verlaufenden parallelen Schlitzen quer zu seiner Längsmittelachse versehen ist, wobei die durch diese Schlitze voneinander getrennten Abschnitte des Zuführkatheters durch wenigstens einen in Längserstreckungsrichtung verlaufenden, mit ihnen verbundenen oder verschweißten Stabilisierungsstreifen verbunden sind, sowie
- Fig. 13: in vergrößertem Maßstab einen Querschnitt des Zuführkatheters gemäß den Fig.11 und 12.

Eine im Ganzen mit 1 bezeichnete Vorrichtung dient zum Einbringen und Platzieren oder Applizieren eines Gegenstands 2, im Ausführungsbeispiel eines Verschlusses für eine nicht gewollte Öffnung 3 zwischen der linken und der rechten Vorhofkammer des Herzens 4, könnte aber auch zum Platzieren oder Applizieren eines anderen Gegenstandes im Inneren eines Blutgefäßes, zum Beispiel zum Zuführen einer Gefäßstütze oder eines mechanischen Blutfilters oder Käfigs oder dergleichen dienen.

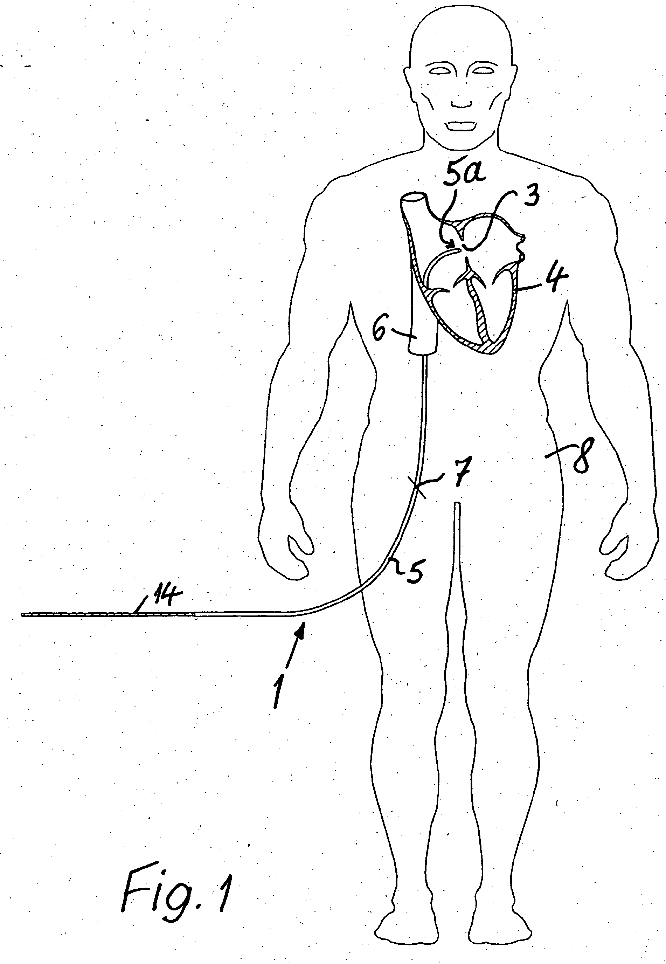

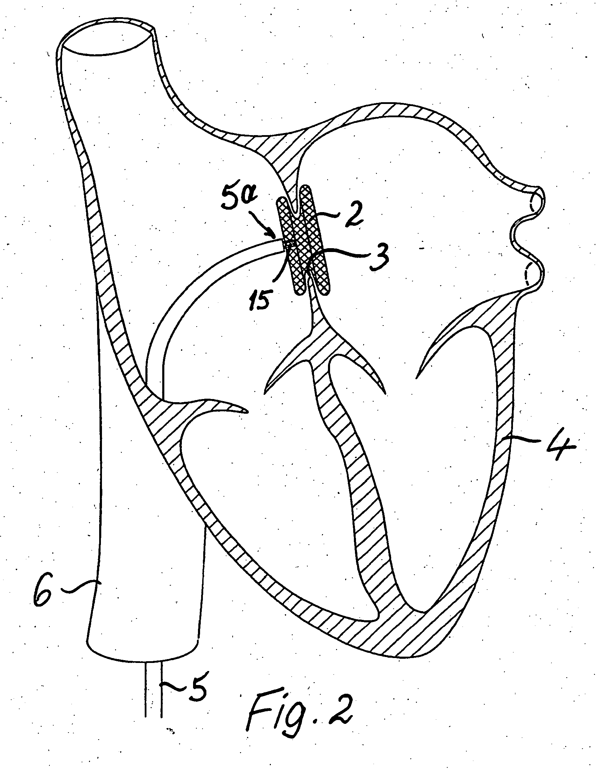

Ein wesentlicher Teil der Vorrichtung 1 ist ein Zuführkatheter 5, der im wesentlichen als Schlauch oder gegebenenfalls als Rohr ausgebildet ist und dessen distales Ende 5a gemäß Fig.2 in Gebrauchsstellung an der Stelle mündet, wo der Gegenstand 2 platziert und appliziert werden soll. Ferner gehört zu der Vorrichtung 1 eine in den Figuren nicht dargestellte Einführvorrichtung, mit welcher der Zuführkatheter 5 von außen in ein zu dem Herzen 4 führendes Blutgefäß 6 einführbar ist. In Fig.1 erkennt man andeutungsweise eine Stelle 7 an einem menschlichen Körper 8, wo der Eintritt des Katheters 5 in ein entsprechendes Blutgefäß 6 erfolgt. In bekannter Weise kann an dieser Stelle eine entsprechende Einführvorrichtung vorgesehen werden.

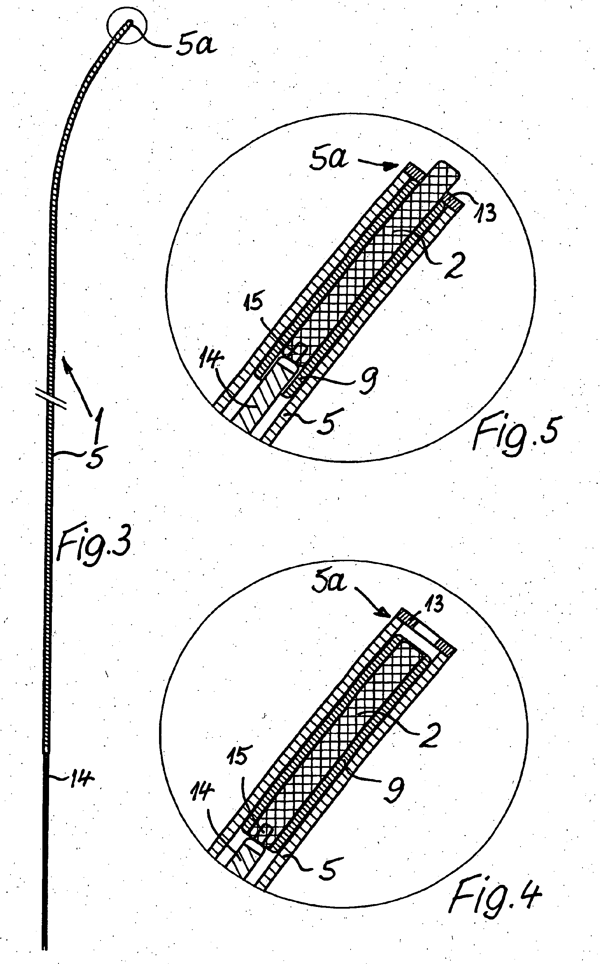

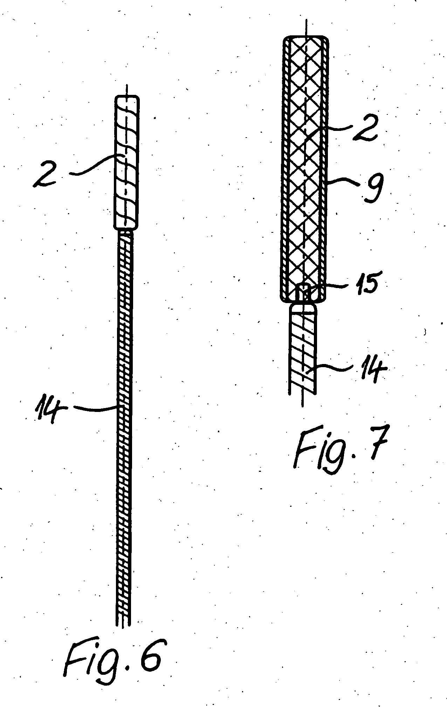

In den Fig.4 und 5 ist dargestellt, dass in dem Katheter 5, im Folgenden auch als "Schlauch 5" bezeichnet, eine Aufnahme 9 für den Gegenstand 2 vorgesehen ist, die diesen Gegenstand 2 vollständig in sich aufnimmt und an den Längsseiten umschließt, wie es die Fig.4 und 7 zeigen.

Gemäß den Fig.4 und 5 ist diese Aufnahme 9 im Inneren des Katheters oder Schlauches 5 bis zu dessen distalem Ende 5a verschiebbar, dass heißt diese Aufnahme 9 dient als Transportmittel oder Transportgefäß für den Gegenstand 2, beispielsweise einen ASD-Verschluss, wie er in Fig.2 dargestellt ist.

An ihrem distalen Ende hat die Aufnahme 9 eine Öffnung oder einen Ausgang zum Ausschieben oder Ausgeben des Gegenstands 2, wie es in Fig.5 dargestellt ist. Somit kann also der Gegenstand 2 innerhalb der Aufnahme 9 durch den Zuführkatheter 5 bis zu dessen distalen Ende 5a hin verschoben werden, ohne selbst mit der Innenseite des Zuführkatheters 5 in Berührung zu kommen. Somit können keine Partikel von dieser Innenseite des Schlauches oder Katheters 5 durch den Gegenstand 2 abgerieben und in die Blutbahn gebracht werden.

Die Aufnahme 9 ist dabei zum Umfassen des Gegenstands 2 als Hülse mit geschlossener Wandung ausgebildet, damit auch ein zusammengedrückter Gegenstand 2 vollständig von der Innenseite des Führungskatheters 5 getrennt bleibt. Diese als Aufnahme 9 dienende Hülse und ihre Innenlängshöhlung dienen also zur Unterbringung des Gegenstands 2 während seines Transports zu dem distalen Ende 5a des Katheters 5 hin.

Der Katheterschlauch 5 kann aus Metall bestehen und flexibel biegsam ausgebildet sein.

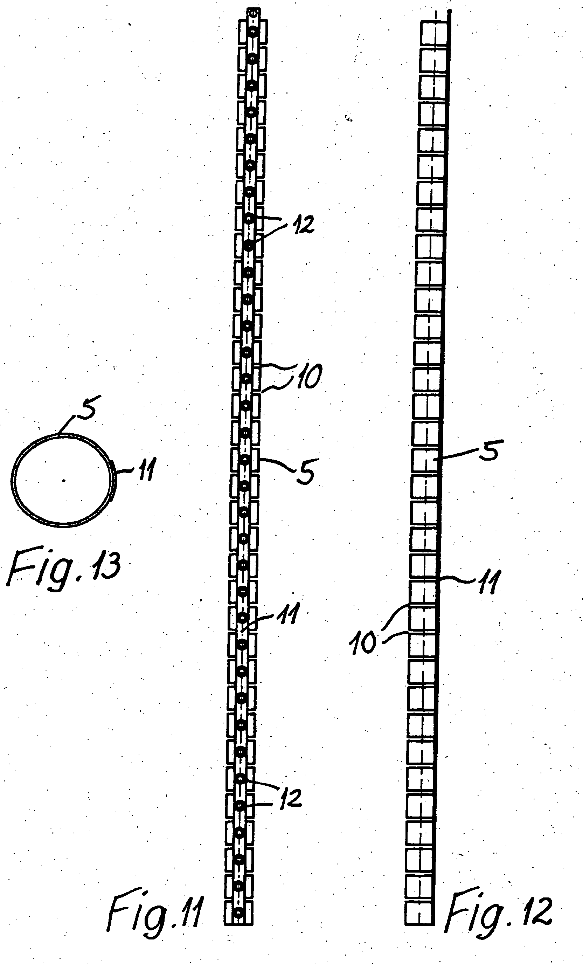

In den Ausführungsbeispielen gemäß Fig.8 bis 13 weist der Katheterschlauch 5 in Umfangsrichtung verlaufende und zueinander beabstandete Schlitze 10 auf, die in beiden Ausführungsbeispielen (Fig.8 bis 10 einerseits und Fig.11 bis 13 andererseits) parallel zueinander angeordnet sind beziehungsweise verlaufen. Dabei erkennt man in Fig.8 und 9, dass diese Schlitze 10 zu einem etwa schraubenlinienförmig verlaufenden Schlitz verbunden sein können, während sie gemäß Fig.11 bis 13 als Einzelschlitze ausgebildet sind. In jedem Falle ergibt sich dadurch eine größere Flexibilität des Katheters oder Schlauches 5 insbesondere, wenn er aus Metall besteht.

Damit dieser geschlitzte Katheter 5 nicht durch die hülsenförmige Aufnahme 9 und deren Verschiebung in Längsrichtung gedehnt werden kann, weist dieser geschlitzte Schlauch 5 in den Ausführungsbeispielen entlang seiner Erstreckung einen Stabilisierungsstreifen 11 insbesondere aus Metall auf, der mit dem Schlauch 5 verbunden, beispielsweise verschweißt ist.

Dabei erkennt man in Fig.8 über die gesamte Länge drei Schweißpunkte 12, während gemäß Fig.11 jeder einzelne Abschnitt des Schlauches 5 mit dem Stabilisierungsstreifen 11 über einen solchen Schweißpunkt 12 verbunden ist, weil die Schlitze 10 über den Gesamtumfang des Katheters 5 verlaufen.

In den Fig.4 und 5 erkennt man, dass am distalen Ende 5a des Katheterschlauches 5 ein sich radial nach innen erstreckender Anschlag 13 für die Aufnahme 9 vorgesehen ist, die gemäß Fig. 5 mit ihrem distalen Ende an dem Anschlag 13 gegen ein Ausschieben aus dem distalen Ende 5a des Katheters 5 gehindert wird, so dass bei einem weiteren Schiebevorgang dann der Gegenstand 2 sowohl aus der Aufnahme 9 als auch aus dem Katheter 5 ausgeschoben werden kann.

Der Anschlag 13 ist dabei als an der Stirnseite des Schlauches oder Katheters 5 umlaufender Ring ausgebildet, dessen Innenöffnung mit dem Innenquerschnitt der hülsenförmigen Aufnahme 2 übereinstimmt, so dass der Gegenstand 2 glatt und ungehindert durch diesen ringförmigen Anschlag 13 hindurch bewegt werden kann. Dabei bildet dieser Anschlag 13 praktisch die Mündung des Schlauches oder Katheters 5 und ist an der Stirnseite des Katheters 5 befestigt.

Zum Verschieben der Aufnahme 9 ist ein an dieser indirekt angreifender Schieber oder Mandrin 14 vorgesehen, der das proximale Ende des Katheters 5 überragt und so eine entsprechende Handhabung und Schiebebewegung relativ zum Katheter 5 ermöglicht. Dabei dient dieser Schieber oder Mandrin 14 oder Pusher auch zum Ausschieben des Gegenstandes 2 aus der Aufnahme 9, nachdem diese den Anschlag 13 erreicht hat, wie es vor allem Fig.5 verdeutlicht.

Dabei ist der Gegenstand 2 gemäß Fig.7 sowie gemäß den Fig.4 und 5 zunächst mit dem Mandrin 14 lösbar verbunden, beispielsweise über eine Gewindeverbindung 15. Dadurch ist es möglich, den Gegenstand 2 gemäß Fig.5 auszuschieben und bis an die Stelle zu bewegen, wo er platziert werden soll, also beispielsweise in eine nicht gewollte Öffnung 3 zwischen der linken und der rechten Vorhofkammer des Herzens 4, wo sich der Gegenstand 2 aus seiner zunächst zusammengedrückten und von der Aufnahme oder Hülse 9 begrenzten Form in seine endgültige gewollte Form als Verschluss entfalten kann, der dann die Öffnung 3 verschließt. Danach kann die Gewindeverbindung 15 zwischen Mandrin 14 und Gegenstand 2 gelöst werden, wenn dieser nämlich seine gewünschte genaue Position hat, so dass dann der Mandrin oder Pusher 14 zurückgezogen werden kann.

Es sei noch erwähnt, dass die als Aufnahme 9 dienende Hülse ihrerseits eine flexible Wandung hat und das beispielsweise der aus Metall bestehende Schlauch oder Katheter 5 und auch diese hülsenförmige Aufnahme 9 jeweils eine Wandstärke von etwas weniger oder etwas mehr oder auch genau von 1/10 mm haben können, was eine platzsparende Anordnung ergibt.

Es wurde schon erwähnt, dass der Mandrin oder Pusher 14 indirekt an der Aufnahme 9 angreifen kann, indem er nämlich an dem in der Aufnahme 9 befindlichen Gegenstand 2 angreift, wobei dennoch zunächst auch die Aufnahme 9 verschoben wird, bis sie den Anschlag 13 erreicht, weil die Reibung zwischen der hülsenförmigen Aufnahme 9 und dem Katheterschlauch 5 geringer ist als die Reibung zwischen dem Gegenstand 2 und dieser Aufnahme 9, in welcher der Gegenstand 2 in der Regel auch unter einem gewissen Druck untergebracht ist.

Denkbar wäre eventuell aber auch, dass unmittelbar an der Aufnahme 9 ein entsprechend gestalteter schlauchförmiger Pusher angreift, in dessen Inneren ein weiterer Pusher oder Mandrin zum Ausschieben des Gegenstands 2 verlaufen könnte.

Die Vorrichtung 1 dient zum Applizieren eines Gegenstandes 2 im Inneren des Körpers, beispielsweise zum Einsetzen eines Verschlusses in eine ungewollte Öffnung 3 zwischen der linken und der rechten Vorhofkammer eines Herzens 4. Dazu weist sie einen Zuführkatheter 5 auf, dessen distales Ende 5a an der Stelle mündet, also zu der Stelle hin verschoben wird, an welcher der Gegenstand 2 platziert oder appliziert werden soll. In üblicher Weise dient eine Einführvorrichtung dazu, den Katheter 5 von außen in ein Blutgefäß 6 einzuführen. Damit beim Verschieben des Gegenstands 2 oder Verschlusses an der Innenseite des Katheters 5 keine Partikel gelöst und in den Blutkreislauf eingetragen werden können, weist die Vorrichtung 1 eine Aufnahme 9 oder Hülse zum Umfassen oder Aufnehmen des Gegenstands 2 auf, worin der Gegenstand 2 durch den Katheter 5 transportiert und geschoben werden kann, ohne mit der Innenseite des Katheters oder Schlauches 5 in Berührung zu gelangen.

## Patentansprüche

1. Vorrichtung (1) zum Einbringen und Platzieren oder Applizieren eines Gegenstandes (2) im Inneren eines Blutgefäßes oder des Herzens, beispielsweise zum Zuführen einer Gefäßstütze (stent) oder eines mechanischen Blutfilters oder Käfigs oder eines Verschlusses für eine nicht gewollte Öffnung (3) zwischen der linken und der rechten Vorhofkammer des Herzens (4), mit einem Zuführkatheter (5), der im wesentlichen als Schlauch ausgebildet ist und dessen distales Ende (5a) in Gebrauchsstellung an der Stelle mündet, wo der Gegenstand (2) platziert werden soll, und mit einer Einführvorrichtung, mit welcher der Katheter (5) von außen in ein Blutgefäß (6) einführbar ist, wobei in dem Katheter eine Aufnahme (9) für den Gegenstand (2) vorgesehen ist, die im Inneren des Katheters oder Schlauches (5) verschiebbar ist, wobei die Aufnahme (9) zum Umfassen des Gegenstandes (2) oder hülsenförmig ausgebildet ist und ihre Innenlängshöhlung zur Unterbringung des Gegenstandes (2) während seines Transports zu dem distalen Ende (5a) des Katheters (5) dient und die Aufnahme (9) an ihrem distalen Ende eine Öffnung oder einen Ausgang zum Ausschieben oder Ausgeben des Gegenstands (2) aus dieser Aufnahme (9) aufweist, **dadurch gekennzeichnet**, das die Aufnahme (9) bis zu dem distalen Ende (5a) des Katheters oder Schlauches (5) verschiebbar ist und dass am distalen Ende (5a) des Katheter oder Schlauches (5) ein sich radial nach innen erstreckender Anschlag (13) für die Aufnahme (9) vorgesehen ist.

2. Vorrichtung nach Anspruch 1, **dadurch gekennzeichnet, dass** der Anschlag (13) als an der Stirnseite oder am Innenumfang des Schlauches oder Katheters (5) umlaufender Vorsprung oder Ring ausgebildet ist.

3. Vorrichtung nach einem der Ansprüche 1 oder 2, **dadurch gekennzeichnet, dass** der Anschlag (13) mit der Mündung des Schlauches oder Katheters (5) bündig ist oder diese Mündung bildet.

4. Vorrichtung nach einem der Ansprüche 1 bis 3, **dadurch gekennzeichnet, dass** der Katheterschlauch (5) aus Metall besteht und flexibel biegsam ausgebildet ist.

5. Vorrichtung nach einem der Ansprüche 1 bis 4, **dadurch gekennzeichnet, dass** der Katheterschlauch (5) in Umfangsrichtung verlaufende und zueinander beabstandete Schlitze (10) aufweist.

6. Vorrichtung nach Anspruch 5, **dadurch gekennzeichnet, dass** die Schlitze (10) die insbesondere parallel zueinander angeordnet sind, wobei sie zu einem etwa schraubenlinienförmig verlaufenden Schlitz verbunden oder als Einzelschlitze ausgebildet sind.

7. Vorrichtung nach einem der Ansprüche 1 bis 6, **dadurch gekennzeichnet, dass** der geschlitzte Schlauch (5) oder Metallschlauch wenigstens einen entlang seiner Erstreckung verlaufenden Stabilisierungsstreifen (11) insbesondere aus Metall aufweist, der mit dem Schlauch (5) verbunden, insbesondere verschweißt ist.

8. Vorrichtung nach einem der Ansprüche 1 bis 7, **dadurch gekennzeichnet, dass** zur Verschiebung der Aufnahme (9) ein an dieser direkt oder indirekt angreifender Schieber oder Mandrin (14) vorgesehen ist und dass dieser Schieber oder Mandrin (14) vorzugsweise auch zum Ausschieben des Gegenstands (2) aus der Aufnahme (9) nach Erreichen des Anschlages (13) dient.

9. Vorrichtung nach einem der Ansprüche 1 bis 8, **dadurch gekennzeichnet, dass** der Mandrin (14) oder Pusher mit dem Gegenstand (2) lösbar verbunden oder verschraubt ist.

10. Vorrichtung nach einem der Ansprüche 1 bis 9, **dadurch gekennzeichnet, dass** die als Aufnahme (9) dienende Hülse eine flexible Wandung hat.

11. Vorrichtung nach einem der Ansprüche 1 bis 10, **dadurch gekennzeichnet, dass** der insbesondere aus Metall bestehende Schlauch (5) des Katheters und/oder die insbesondere hülsenförmige Aufnahme (9) eine Wandstärke von etwa 1/10 mm oder weniger oder etwas mehr hat.

12. Vorrichtung nach einem der Ansprüche 1 bis 11, **dadurch gekennzeichnet, dass** die Reibung zwischen der insbesondere hülsenförmigen Aufnahme (9) und dem KatheterSchlauch (5) geringer als zwischen dem Gegenstand (2) und der Aufnahme (9) ist.

## Claims

1. Device (1) for introducing and placing or applying an object (2) inside a blood vessel or the heart, for example for introducing a stent or a mechanical blood filter or cage or a closure for an unwanted opening (3) between the left and right atrium of the heart (4), with a delivery catheter (5) that is formed substantially as a tube and the distal end (5a) of which terminates, in the position of use, at the point where the object (2) is to be placed, and with an introducing device with which the catheter (5) can be inserted from outside into a blood vessel (6), while in the catheter is provided a receptacle (9) for the object (2), said receptacle being movable inside the catheter or tube (5), the receptacle (9) being designed to surround the object (2) or sleeve-shaped, and its inner longitudinal lumen serves to accommodate the object (2) during its transportation to the distal end (5a) of the catheter (5) and the receptacle (9) comprises at its distal end an opening or an exit for pushing out or delivering the object (2) from this receptacle (9), **characterised in that** the receptacle (9) can be pushed up to the distal end (5a) of the catheter or tube (5) and that a radially inwardly extending stop (13) is provided for the receptacle (9) at the distal end (5a) of the catheter or tube (5).

2. Device according to claim 1, **characterised in that** the stop (13) is constructed as a projection or ring running in a circle around the end face or the inner circumference of the tube or catheter (5).

3. Device according to claim 1 or 2, **characterised in that** the stop (13) is flush with the opening of the tube or catheter (5) or forms this opening.

4. Device according to one of claims 1 to 3, **characterised in that** the catheter tube (5) is made of metal and is flexibly bendable in design.

5. Device according to one of claims 1 to 4, **characterised in that** the catheter tube (5) has slots (10) extending in the circumferential direction at a spacing from one another.

6. Device according to claim 5, **characterised in that** the slots (10), which are arranged parallel to one another, in particular, are joined together to form a substantially helically extending slot or are in the form of individual slots.

7. Device according to one of claims 1 to 6, **characterised in that** the slotted tube (5) or metal tube comprises at least one stabilising strip (11) extending along its length, this strip being made of metal, in particular, and connected to the tube (5), particularly by welding.

8. Device according to one of claims 1 to 7, **characterised in that** for moving the receptacle (9) a pusher or mandrel (14) is provided which acts directly or indirectly thereon, and **in that** this pusher or mandrel (14) preferably also serves to push the object (2) out of the receptacle (9) after reaching the stop (13).

9. Device according to one of claims 1 to 8, **characterised in that** the mandrel (14) or pusher is releasably connected or screwed to the object (2).

10. Device according to one of claims 1 to 9, **characterised in that** the sleeve acting as the receptacle (9) has a flexible wall.

11. Device according to one of claims 1 to 10, **characterised in that** the tube (5) of the catheter, which is made of metal, in particular, and/or the in particular sleeve-shaped receptacle (9) has a wall thickness of about 1/10 mm or less or somewhat more.

12. Device according to one of claims 1 to 11, **characterised in that** the friction between the in particular sleeve-shaped receptacle (9) and the catheter tube (5) is less than between the object (2) and the receptacle (9).

## Revendications

1. Dispositif (1) pour introduire et mettre en place ou appliquer un objet (2) à l'intérieur d'un vaisseau sanguin ou du coeur, par exemple pour amener un soutien de vaisseau (« stent ») ou un filtre sanguin mécanique ou une cage ou un obturateur pour une ouverture indésirable (3) entre l'oreillette gauche et l'oreillette droite du coeur (4), avec un cathéter d'amenée (5) qui est réalisé essentiellement sous forme de tuyau souple et dont l'extrémité distale (5a) débouche, en position d'utilisation, à l'endroit où doit être placé l'objet (2), et avec un dispositif d'introduction par lequel le cathéter (5) peut être introduit de l'extérieur dans un vaisseau sanguin (6), sachant qu'un réceptacle (9) pour l'objet (2) est prévu dans le cathéter, réceptacle qui peut être coulissé à l'intérieur du cathéter ou tuyau souple (5), sachant que le réceptacle (9) est conçu pour embrasser l'objet (2) ou en forme de manchon et sa cavité longitudinale intérieure sert à loger l'objet (2) pendant son transport vers l'extrémité distale (5a) du cathéter (5), et le réceptacle (9) présente à son extrémité distale une ouverture ou une sortie pour pousser ou évacuer l'objet (2) hors de ce réceptacle (9), **caractérisé en ce que** le réceptacle (9) peut être coulissé jusqu'à l'extrémité distale (5a) du cathéter ou tuyau souple (5), et **en ce qu'**une butée (13) pour le réceptacle (9), laquelle s'étend radialement vers l'intérieur, est prévue à l'extrémité distale (5a) du cathéter ou tuyau souple (5).

2. Dispositif selon la revendication 1, **caractérisé en ce que** la butée (13) est réalisée sous la forme d'une saillie ou d'un anneau entourant(e) sur la face frontale ou sur la circonférence intérieure du tuyau souple ou cathéter (5).

3. Dispositif selon la revendication 1 ou 2, **caractérisé en ce que** la butée (13) est à fleur de l'embouchure du tuyau souple ou cathéter (5), ou forme cette embouchure.

4. Dispositif selon l'une des revendications 1 à 3, **caractérisé en ce que** le tuyau souple de cathéter (5) est réalisé en métal et est réalisé souple et flexible.

5. Dispositif selon l'une des revendications 1 à 4, **caractérisé en ce que** le tuyau souple de cathéter (5) présente des fentes (10) s'étendant en direction circonférentielle et mutuellement distantes.

6. Dispositif selon la revendication 5, **caractérisé en ce que** les fentes (10) sont notamment disposées parallèlement entre elles, sachant qu'elles sont réunies en une fente s'étendant approximativement en hélice ou qu'elles sont réalisées sous forme de fentes individuelles.

7. Dispositif selon l'une des revendications 1 à 6, **caractérisé en ce que** le tuyau souple métallique ou tuyau souple (5) fendu présente au moins une bande de stabilisation (11) s'étendant le long de son étendue, notamment en métal, qui est assemblée au tuyau souple (5), notamment par soudage.

8. Dispositif selon l'une des revendications 1 à 7, **caractérisé en ce qu'**un coulisseau ou mandrin (14) agissant directement ou indirectement sur le réceptacle (9) est prévu pour faire coulisser ce dernier, et **en ce que** ce coulisseau ou mandrin (14) sert de préférence également à pousser l'objet (2) hors du réceptacle (9) suite à l'atteinte de la butée (13).

9. Dispositif selon l'une des revendications 1 à 8, **caractérisé en ce que** le mandrin (14) ou pousseur est relié par vissage ou de manière détachable à l'objet (2).

10. Dispositif selon l'une des revendications 1 à 9, **caractérisé en ce que** le manchon servant de réceptacle (9) possède une paroi flexible.

11. Dispositif selon l'une des revendications 1 à 10, **caractérisé en ce que** le tuyau souple (5), réalisé notamment en métal, du cathéter et/ou le réceptacle (9) notamment en forme de manchon possèdent une épaisseur d'environ 1/10 mm ou moins ou un peu plus.

12. Dispositif selon l'une des revendications 1 à 11, **caractérisé en ce que** le frottement entre le réceptacle (9) notamment en forme de manchon et le tuyau souple de cathéter (5) est moindre qu'entre l'objet (2) et le réceptacle (9).
